(19) Europäisches Patentamt
European Patent Office
Office européen des brevets

(11) **EP 3 283 878 B1**

(12) **FASCICULE DE BREVET EUROPEEN**

(45) Date de publication et mention
de la délivrance du brevet:
**11.12.2019 Bulletin 2019/50**

(51) Int Cl.:
***G01N 33/24*** *(2006.01)*   ***E02D 1/02*** *(2006.01)*

(21) Numéro de dépôt: **16733129.7**

(22) Date de dépôt: **14.04.2016**

(86) Numéro de dépôt international:
**PCT/FR2016/050860**

(87) Numéro de publication internationale:
**WO 2016/166477 (20.10.2016 Gazette 2016/42)**

(54) **PROCÉDÉ DE DÉTERMINATION DE LA PRESSION INTERSTITIELLE DANS LE SEDIMENT MARIN ET DISPOSITIF CORRESPONDANT**

VERFAHREN ZUR BESTIMMUNG DES INTERSTITIELLEN DRUCKS IM MEERESSEDIMENT UND ENTSPRECHENDE VORRICHTUNG

METHOD OF DETERMINING THE INTERSTITIAL PRESSURE IN THE MARINE SEDIMENT AND CORRESPONDING DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.04.2015 FR 1553355**

(43) Date de publication de la demande:
**21.02.2018 Bulletin 2018/08**

(73) Titulaire: **Institut Français de Recherche pour l'Exploitation de la Mer (IFREMER)
92138 Issy-les-Moulineaux Cedex (FR)**

(72) Inventeurs:
• **WOERTHER, Patrice**
  **29280 Plouzane (FR)**
• **JEGOU, Paul**
  **29200 Brest (FR)**
• **GUILLEMOT, Anne**
  **29810 Plouarzel (FR)**
• **LE VOURC'H, Damien**
  **29870 Lannilis (FR)**
• **LE PIVER, David**
  **29200 Brest (FR)**
• **FERRANT, Antony**
  **29810 Plouarzel (FR)**
• **COAIL, Jean-Yves**
  **29280 Plouzane (FR)**
• **ROUDAUT, Mickael**
  **29890 Plouneour Trez (FR)**

(74) Mandataire: **Novagraaf Technologies
12 place des Halles Saint Louis
56100 Lorient (FR)**

(56) Documents cités:
**WO-A1-2014/131085    US-A- 4 150 578
US-A- 5 804 715    US-A1- 2004 118 199**

• **Nke Instrumentation: "Piezometer V2", , 25 octobre 2010 (2010-10-25), XP055297965, Extrait de l'Internet: URL:http://www.nke-instrumentation.com/media/pdf/file3/nke-piezometer-light-10-1290591698.pdf [extrait le 2016-08-26]**
• **JACQUES MEUNIER ET AL: "First Tests of Penfeld : a New Seabed Penetrometer", THE FOURTEENTH INTERNATIONAL OFFSHORE AND POLAR ENGINEERING CONFERENCE, 28 mai 2004 (2004-05-28), pages 338-345, XP055248291,**
• **Paolo Favali ET AL: "Seafloor observations and observatory activities in the Sea of Marmara" In: "SEAFLOOR OBSERVATORIES", 1 janvier 2015 (2015-01-01), Springer Berlin Heidelberg, Berlin, Heidelberg, XP055248969, ISBN: 978-3-642-11374-1 pages 59-79, DOI: 10.1007/978-3-642-11374-1_4, figure 4.6**

EP 3 283 878 B1

**Description**

**Domaine technique**

La présente invention se rapporte au domaine de la géotechnique pour la mesure de la pression interstitielle dans le sédiment marin. L'invention concerne un procédé de détermination de la pression interstitielle dans le sédiment marin à différents niveaux de profondeur et un dispositif pour mettre en œuvre ledit procédé.

**Etat de la technique**

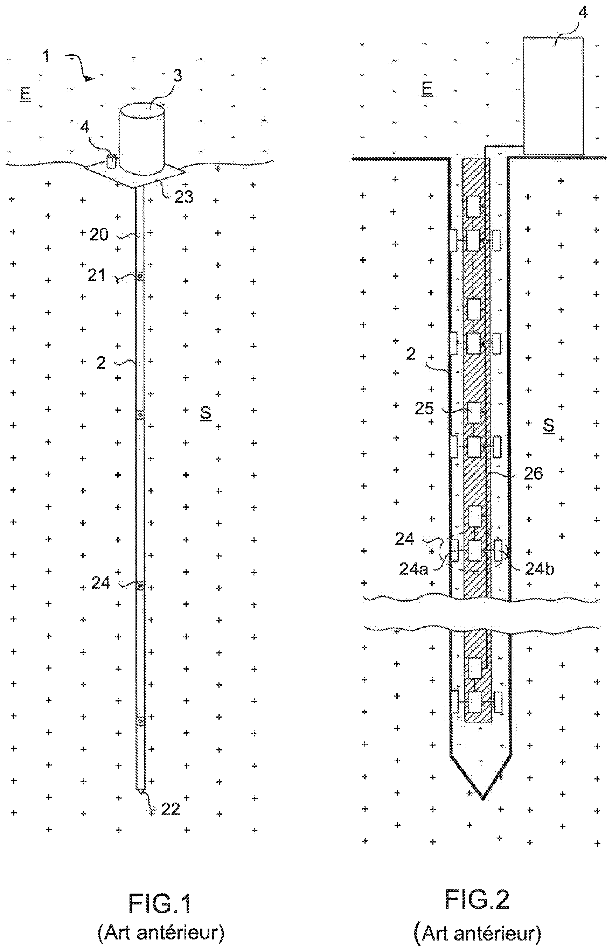

Il existe actuellement des dispositifs de mesure de pression interstitielle in-situ dans le sédiment marin. On connaît notamment le dispositif, appelé piézomètre V2, développé par l'IFREMER pour mesurer la pression interstitielle et la température dans le sédiment marin à des profondeurs allant jusqu'à 6000 mètres. Un tel dispositif est représenté schématiquement aux figures 1 à 3. Les figures étant des représentations schématiques, les proportions des éléments du dispositif ne sont pas nécessairement respectées.

En référence aux figures 1 et 2, ce dispositif, référencé 1 dans la figure 1, comprend essentiellement une tige instrumentée perdable 2 et un lest 3 et un module électronique 4 tous deux récupérables. Le module électronique et le lest sont disposés à l'extrémité supérieure de la tige. La tige 2 est équipée d'une pluralité de capteurs de pression associés à des cartes électroniques d'acquisition des mesures et est destinée à être enfoncée dans le sédiment S de façon gravitaire au moyen du lest 3.

Le module électronique 4 comprend des moyens de stockage pour stocker les mesures de pression, des moyens d'alimentation pour alimenter en énergie les cartes électroniques associées aux capteurs de pression, un largueur acoustique pour libérer le module électronique et des moyens pour le faire remonter à la surface après la phase de mesure.

Des moyens de largage (non représentés) sont également prévus au niveau du lest 3 pour le déconnecter de la tige après le "planté" de cette dernière dans le sédiment.

La tige 2 perdable comprend une pluralité de tronçons de tube 20 reliés entre eux par des éléments de liaison 21, un cône de pénétration 22 à son extrémité inférieure pour faciliter l'enfoncement de la tige dans le sédiment et une jupe 23 dans sa partie supérieure pour éviter que la tige ne s'enfonce entièrement dans le sédiment.

La tige 2 est par ailleurs équipée de capteurs de pression 24 disposés au niveau des éléments de liaison 21, de cartes électroniques associées 25 pour l'acquisition des résultats de mesure issus des capteurs de pression, d'un bus numérique de communication 26 pour relier les cartes électroniques 25 au module électronique 4. Les tronçons de tube et les éléments de liaison sont connectés entre eux par vissage ou tout autre moyen de liaison.

La tige est réalisée en un matériau rigide pour éviter qu'elle ne se déforme au moment de son enfoncement dans le sédiment. Les capteurs de pression 24 sont placés à intervalles prédéfinis, au niveau des éléments de liaison 21, le long de la tige. Ce sont des capteurs différentiels qui mesurent la différence entre la pression hydrostatique d'une colonne d'eau reliée à l'interface eau/sédiment et la pression dans le sédiment au niveau du capteur. A cet effet, l'extrémité supérieure de la tige 2 est ouverte sur le milieu extérieur, à savoir le milieu marin, pour que l'eau puisse parvenir jusqu'aux capteurs de pression.

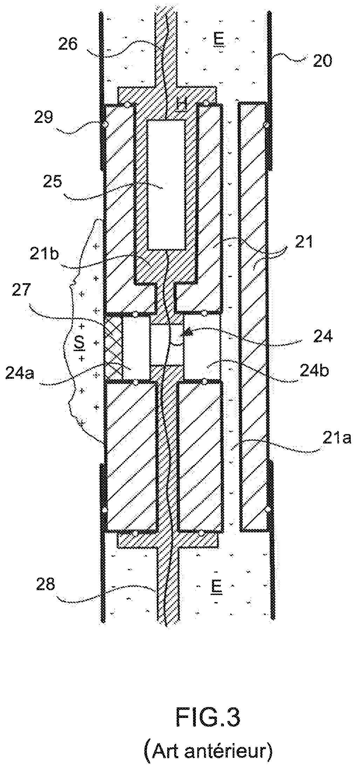

Une vue schématique partielle en coupe de la tige au niveau d'un élément de liaison 21 est montrée à la figure 3. L'élément de liaison 21 est représenté en trait hachuré. Il comprend une cavité débouchant sur le milieu extérieur pour recevoir le capteur de pression 24. Ce capteur comprend deux éléments sensibles 24a et 24b à la pression à ses deux extrémités. Lorsque la tige est enfoncée dans le sédiment, l'élément sensible 24a est en contact avec le sédiment via une pierre poreuse 27. L'autre élément sensible, 24b, est en contact avec une colonne d'eau reliée à l'interface eau/sédiment (extrémité supérieure ouverte de la tige). Pour assurer la continuité de la colonne d'eau entre l'extrémité ouverte de la tige (présente au niveau de l'interface eau/sédiment) et l'élément sensible 24b, l'élément de liaison 21 est pourvu d'un canal interne 21a traversant de part en part dans le sens vertical l'élément de liaison. L'élément sensible 24b est en contact avec l'eau présente dans ce canal interne.

Par ailleurs, tous les circuits électroniques à l'intérieur de la tige, à savoir les cartes électroniques 25, la partie électronique des capteurs 24 et le bus numérique de communication 26, sont isolés de la partie de la tige remplie d'eau et sont disposés dans une partie remplie d'huile. A cet effet, l'élément de liaison 21 comporte un autre canal interne, référencé 21b, traversant de part en part l'élément de liaison et dans lequel sont disposés la partie électronique du capteur de pression 24, la carte électronique 25 associée au capteur et le bus numérique de communication 26. Les canaux internes 21b des éléments de liaison 21 successifs sont connectés entre eux par des tuyaux souples 28 remplis d'huile. Des joints toriques 29 sont prévus à divers endroits de l'élément de liaison 21 pour assurer l'étanchéité entre le sédiment et l'intérieur de la tige ainsi que l'étanchéité entre la partie en huile et la partie en eau.

Avec ce dispositif, le capteur de pression mesure la différence entre la pression hydrostatique d'une colonne d'eau reliée à l'interface eau/sédiment (pression captée par l'élément sensible 24b) et la pression dans le sédiment au niveau du capteur (pression captée par l'élément sensible 24a). Cette mesure est transmise au module électronique 4 via la carte électronique 25 et le bus numérique de communication 26.

Ce dispositif présente les inconvénients suivants. La longueur de la tige de ce dispositif est actuellement limitée à 15 mètres en raison des contraintes de taille des apparaux de mise à l'eau et de récupération du dispositif. On est donc limité à 6 ou 10 capteurs disposés le long de la tige.

Par ailleurs, ce dispositif nécessite un diamètre de tige suffisamment grand pour recevoir les capteurs de pression différentielle, les cartes électroniques, le bus numérique de communication et les deux réseaux de liquide, à savoir le réseau d'eau renfermant la colonne d'eau nécessaire à la mesure de pression différentielle et un réseau d'huile pour l'électronique du système.

## Résumé de l'invention

Un objet de l'invention est de proposer un procédé et un dispositif de mesure de pression interstitielle qui permette d'effectuer des mesures de pression à de plus grandes profondeurs dans le sédiment tout en ayant un encombrement réduit au moment de sa mise à l'eau.

A cet effet, l'invention concerne un procédé de détermination de la pression interstitielle dans le sédiment marin à différentes profondeurs, ledit procédé comportant les étapes suivantes:

- enfoncer dans le sédiment marin une tige creuse équipée d'une pluralité de capteurs de pression et de capteurs de température disposés le long de la tige, ladite tige étant remplie de liquide diélectrique,

- mesurer au moyen des capteurs de pression la pression dans le sédiment par rapport à la pression du liquide diélectrique dans la tige pour une pluralité de niveaux de profondeur,
- déterminer au moyen des capteurs de température la température à proximité des capteurs de pression, et
- déterminer une valeur réelle de pression interstitielle pour chacun des niveaux de profondeur à partir des mesures de pression fournies par les capteurs de pression et les mesures de température fournies par les capteurs de température.

Ainsi, selon l'invention, la tige est remplie de liquide diélectrique qui peut être de l'huile, les capteurs de pression sont des capteurs relatifs aptes à mesurer chacun la pression dans le sédiment par rapport à la pression du liquide diélectrique dans la tige pour un niveau de profondeur donné et les capteurs de température sont aptes à mesurer la température du liquide diélectrique à proximité des capteurs de pression. Cela permet d'utiliser des capteurs avec un encombrement réduit et de supprimer la partie remplie d'eau (réseau d'eau) des dispositifs de l'art antérieur. La mesure de pression n'est plus réalisée par une mesure différentielle entre la pression dans le sédiment et la pression d'une colonne d'eau comme dans les dispositifs de l'art antérieur. La suppression de la partie en eau (ou réseau d'eau) dans la tige et l'utilisation de capteurs de pression relatifs permet de réduire le diamètre de la tige et de faciliter son cintrage et son décintrage. La mesure de température permet de corriger la mesure de pression relative ou de calculer les flux de chaleur.

Selon un mode de réalisation particulier, les capteurs de température sont présents à l'intérieur de la tige pour mesurer la température du liquide diélectrique au niveau de chaque capteur de pression.

Selon un mode de réalisation particulier, la tige est enfoncée dans le sédiment marin à l'aide d'un pénétromètre à tambour, ladite tige étant enroulée par cintrage autour du tambour avant enfoncement. Une fois posé sur le fond marin, la tige est déroulée puis redressée mécaniquement par le pénétromètre avant d'être enfoncée dans le sédiment.

Selon un mode de réalisation particulier, le liquide diélectrique est de l'huile.

L'invention concerne également un dispositif de mesure de pression interstitielle dans le sédiment marin comprenant:

- une tige creuse remplie de liquide diélectrique et destinée à être enfoncée dans le sédiment marin,
- une pluralité de capteurs de pression disposés le long de la tige pour mesurer la pression interstitielle dans le sédiment marin à différentes profondeurs,
- une pluralité de capteurs de température disposés à proximité des capteurs de pression pour mesurer la température à proximité des capteurs de pression,
- une pluralité de cartes d'acquisition des mesures connectées chacune à un capteur de pression et à un capteur de température, et un bus numérique de communication pour transmettre les données de mesure vers l'extérieur de la tige, ladite pluralité de cartes d'acquisition de mesure et le bus numérique de communication étant disposés à l'intérieur de la tige remplie de liquide diélectrique,

dans lequel les capteurs de pression sont des capteurs relatifs et sont agencés dans la tige pour chacun mesurer, pour une profondeur donnée, la pression dans le sédiment par rapport à la pression du liquide diélectrique dans la tige.

Selon un mode de réalisation particulier, les capteurs de température sont présents à l'intérieur de la tige pour mesurer la température du liquide diélectrique au niveau de chaque capteur de pression.

Selon un mode de réalisation particulier, la tige comporte une pluralité de tronçons (120) assemblés entre eux par des éléments de liaison (121) étanches.

Selon un mode de réalisation particulier, les tronçons de tube et les éléments de liaison sont réalisés en un matériau ductile, tel que de l'acier inoxydable X2CrNiMo 17-12-2.

Selon un mode de réalisation particulier, la tige comporte un capteur de pression et un capteur de température par tronçon de tube ou par élément de liaison.

Selon un mode de réalisation particulier, le liquide diélectrique à l'intérieur de la tige est de l'huile. L'invention concerne également un système de mesure de pression interstitielle dans le sédiment marin à différents niveaux de profondeur, comprenant un dispositif tel que défini précédemment et un pénétromètre pour enfoncer la tige dans le sédiment marin, ledit pénétromètre comprenant un tambour autour duquel est enroulée la tige avant enfoncement et des moyens d'enfoncement de la tige dans le sédiment marin, la tige étant apte à être au moins une fois enroulée par cintrage autour du tambour et déroulée du tambour et lesdits moyens d'enfoncement étant aptes à dérouler et redresser mécaniquement la tige du tambour puis l'enfoncer dans le sédiment marin.

L'utilisation d'un pénétromètre à tambour et d'une tige apte être enroulée par cintrage autour de ce tambour puis déroulée par décintrage permet d'atteindre de très grandes profondeurs dans le sédiment tout en ayant un encombrement réduit au moment de la mise à l'eau du système.

D'autres avantages pourront encore apparaître à l'homme du métier à la lecture des exemples ci-dessous, illustrés par les figures annexées, donnés à titre illustratif.

**Brève description des figures**

- La figure 1, déjà décrite, est une vue d'ensemble d'un système de mesure de pression interstitielle dans le sédiment marin selon l'art antérieur;

- La figure 2, déjà décrite, est une vue schématique en coupe de la tige équipée de capteurs de pression du système de la figure 1;

- La figure 3, déjà décrite, est une vue en coupe détaillée de la tige du système des figures 1 et 2 au niveau d'un élément de liaison de la tige;

- La figure 4 est une vue d'ensemble d'un système de mesure de pression interstitielle dans le sédiment marin selon l'invention, avant enfoncement de la tige dans le sédiment;

- La figure 5 est une vue d'ensemble du système de la figure 4 après enfoncement de la tige dans le sédiment;

- La figure 6 est une vue schématique en coupe de la tige équipée de capteurs de pression du système des figures 4 et 5; et

- La figure 7 est une vue en coupe détaillée de la tige du système des figures 4 et 5 au niveau d'un élément de liaison de la tige.

**Description détaillée de l'invention**

Selon l'invention, la tige instrumentée du système de mesure de pression interstitielle est conçue pour être enfoncée dans le sédiment à l'aide d'un pénétromètre à tambour, tel que le pénétromètre PENFELD. A cet effet, les tronçons de tube et les éléments de liaison de la tige sont réalisées en un matériau ductile de manière à ce que la tige puisse être cintrée puis enroulée autour du tambour du pénétromètre. Par ailleurs, les capteurs de pression employés sont des capteurs de mesure relative aptes à mesurer la pression dans le sédiment par rapport à la pression dans une partie de la tige remplie d'un liquide diélectrique tel que de l'huile.

Nous allons tout d'abord décrire ce qu'est un pénétromètre à tambour, et plus particulièrement le pénétromètre Penfeld. Un tel pénétromètre est par exemple décrit dans l'article intitulé "Le pénétromètre Penfeld" de J.Meunier, N. Sultan, P.Jégou, F.Harmegnies, SeatechWeek 2004, caractérisation des fonds, 21-22 octobre 2004.

Le pénétromètre Penfeld est un équipement lourd développé au début des années 2000 par Ifremer pour effectuer des mesures géotechniques sur les fonds sédimentaires immergés allant des zones côtières peu profondes aux plaines abyssales de 6000 mètres de fond. C'est un engin opérationnel d'investigation géotechnique des sols sous-marins capable d'enfoncer à une vitesse constante une tige continue de 36 mm de diamètre extérieur.

Le pénétromètre est déposé sur le fond marin à l'aide d'un câble d'un navire et y reste pendant toute la durée des opérations de mesure géotechnique ou de déploiement de la tige instrumentée. Il est habituellement utilisé pour enfoncer dans le sédiment une tige, d'un seul tenant, muni au niveau de son extrémité inférieure (ou pointe) d'un ou plusieurs capteurs. La tige est stockée enroulée sur un tambour de 2,25 m de diamètre, ce qui permet d'obtenir un engin dont l'encombrement est réduit et adapté aux conditions de transport terrestre et maritime. La tige est enroulée autour du tambour selon la méthode du «coiled tubing» qui consiste à cintrer la tige par déformation plastique. Pour son enfoncement dans le sédiment, la tige est redressée plastiquement puis poussée dans le sol.

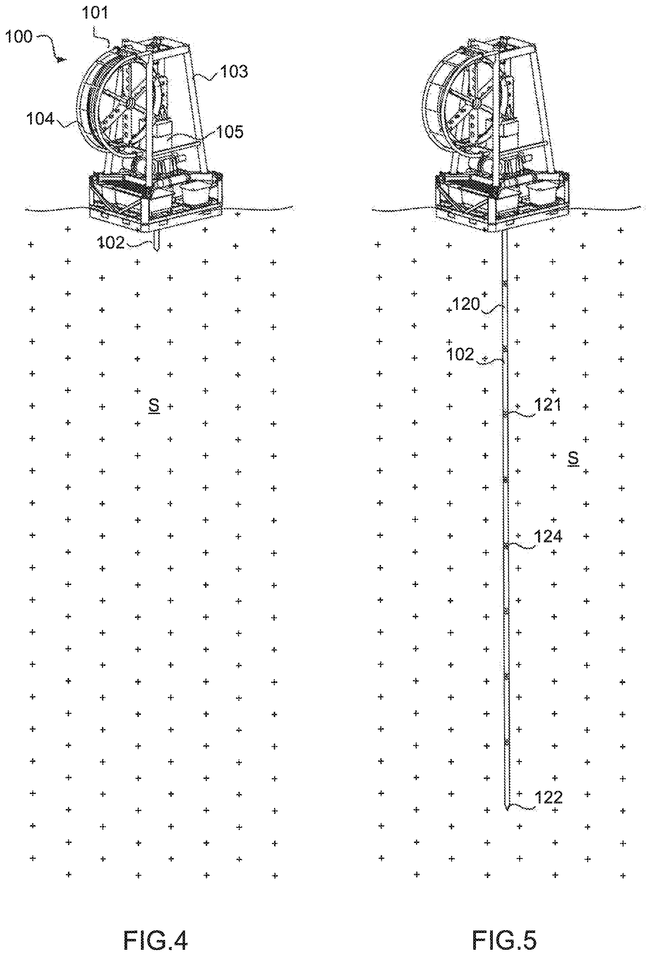

En référence à la figure 4, le pénétromètre, référencé 100, comporte sommairement un châssis 101 supportant les différents éléments du pénétromètre et destiné à être posé sur le fond marin, un tambour 104 comprenant un axe cylindrique autour duquel est enroulée la tige 102 par cintrage et un moyen d'enfoncement 105 de la tige, appelé «injecteur», qui enserre la tige entre deux chaînes entrainées par un moteur, la déroule du tambour, la redresse au moyen de galets et l'enfonce dans le sédiment marin.

Le poids de l'engin dans l'air est de 93 kN. Son poids dans l'eau est de 65 kN. La réaction du sol est limitée à 50 kN.

Selon l'invention, ce pénétromètre est utilisé pour enfoncer une tige formée d'une pluralité de tronçons de tube assemblés entre eux par des éléments de liaison et équipée d'une pluralité de capteurs de pression et de température disposés le long de celle-ci. Le pénétromètre étant connu pour enfoncer une tige d'un seul tenant ayant des capteurs uniquement au niveau de sa pointe, l'invention a consisté à réaliser une tige tel que précitée (formée de plusieurs tronçons de tube) qui puisse supporter au moins une opération de cintrage et une opération de décintrage sans endommager les capteurs et sans dégrader la tenue mécanique et l'étanchéité de la tige. L'invention a également consisté à réaliser une tige ayant un diamètre identique à celui des tiges enfoncées habituellement par le pénétromètre Penfeld, à savoir un diamètre de l'ordre de 36 mm, pour ne pas avoir à modifier ce dernier. Cette réduction de diamètre de la tige a notamment été obtenue par l'utilisation de capteurs de mesure de pression relative plus compacts que les capteurs de mesure de pression différentielle habituellement employés.

Un système de mesure de pression interstitielle conforme à l'invention est décrit en référence aux figures 4 à 7.

La figure 4, déjà décrite partiellement ci-dessus, est une vue d'ensemble d'un système de mesure de pression interstitielle conforme à l'invention avant enfoncement de la tige 102 dans le sédiment. La tige est alors enroulée autour du tambour 103 du pénétromètre. La figure 5 représente la même vue après enfoncement de la tige 102 dans le sédiment marin.

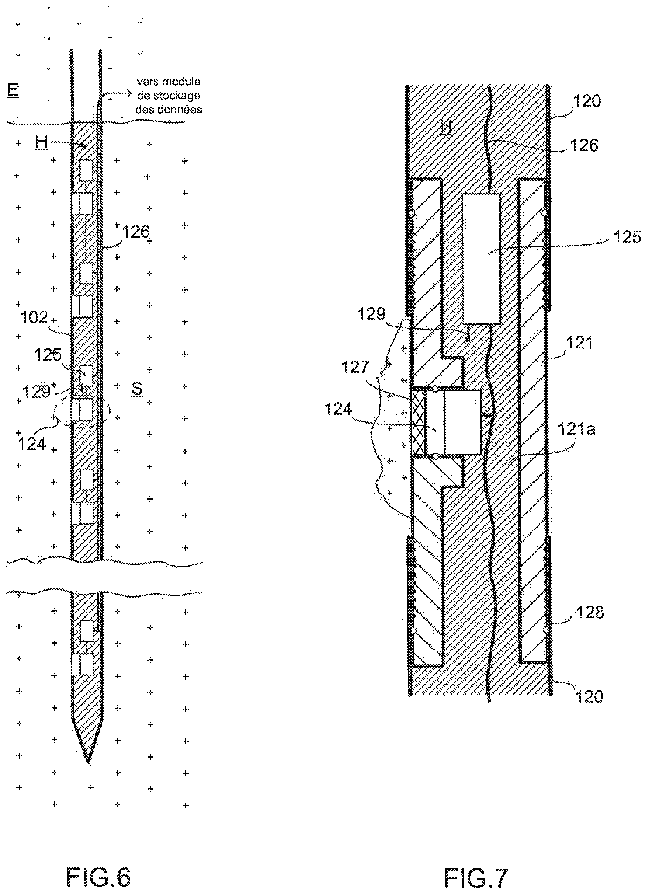

La tige 102 est composée d'une pluralité de tronçons de tube 120 assemblés entre eux par des éléments de liaison 121 visibles sur les figures 4 et 5. Elle comporte une pointe 122 à son extrémité inférieure. Les tronçons de tube 120 sont connectés aux éléments de liaison 121 par vissage. A cet effet, les extrémités de tronçon de tube à assembler comportent un filetage intérieur et les extrémités des éléments de liaison un filetage extérieur (filetages visibles sur la figure 7).

La tige est équipée de capteurs de pression 124 répartis le long de la tige. Dans l'exemple présenté, les capteurs 124 sont présents au niveau des éléments de liaison 121. Selon une variante, les capteurs peuvent être disposés au niveau de tronçons de tube. La tige comporte également des capteurs de température 129, un capteur de température 129 étant disposé à proximité de chacun des capteurs de pression 124.

La figure 6 est une vue schématique en coupe de la tige 102 selon l'invention et la figure 7 est une vue partielle plus détaillée de cette tige. La tige creuse est remplie d'un liquide diélectrique tel que de l'huile H. Chaque capteur de pression 124 et chaque capteur de température 129 est relié à une carte électronique d'acquisition des mesures 125. Dans les figures 6 et 7, la carte électronique 125 étant disposée à proximité du capteur de pression 124, le capteur de température 126 est monté directement sur la carte électronique 125.

Les cartes électroniques 125 sont reliées à un module de stockage des données (extérieur à la tige) par un bus numérique de communication 126.

Les cartes électroniques 125 et le bus numérique de communication sont présents dans la partie en huile de la tige.

Selon un premier aspect important de l'invention, les tronçons de tube 120 et les éléments de liaison 121 sont réalisés en un matériau ductile de manière à ce que la tige puisse être enroulée autour du tambour du pénétromètre et puisse être déroulée pendant la phase de pénétration dans le sédiment S. Lors des opérations de cintrage et de décintrage, le filetage des tronçons et des éléments de liaison peut se déformer. Cependant l'utilisation d'un matériau ductile permet à la tige de conserver une bonne tenue mécanique et une parfaite étanchéité. Selon un mode de réalisation particulier, les tronçons de tube 120 et les éléments de liaison 121 sont réalisés en acier inoxydable X2CrNiMo 17-12-2 (316L).

Selon un autre aspect important de l'invention, les capteurs 124 sont des capteurs de mesure de pression relative aptes à mesurer, pour un niveau de profondeur donné, la pression dans le sédiment par rapport à la pression de l'huile dans la tige. Chacun des capteurs est disposé dans une cavité transversale de l'élément de liaison 121 de pouvoir à être en contact, d'un coté, avec le sédiment S au travers d'une pierre poreuse 127, et, de l'autre coté, avec l'huile H présente dans la tige. A cet effet, l'élément de liaison comporte un canal interne, référencé 121a, traversant verticalement et de part en part l'élément de liaison et dans lequel sont disposés la partie électronique du capteur de pression 124, la carte électronique 125, le capteur de température 129 et le bus numérique de communication 126.

Des joints toriques 128 sont prévus à divers endroits de l'élément de liaison 121 pour assurer l'étanchéité entre le sédiment et l'intérieur de la tige.

Les capteurs 124 mesurent la pression dans le sédiment par rapport à celle exercée par l'huile H sur le capteur. L'huile utilisée est par exemple une huile minérale.

Une fois cette mesure effectuée, il faut y apporter une correction pour obtenir la valeur réelle de la pression interstitielle dans le sédiment. Cette correction est fonction de la température du milieu au niveau du capteur. Selon un mode de réalisation particulier de l'invention, on mesure la température de l'huile à l'intérieur de la tige au niveau du capteur. A cet effet, la carte électronique 125 est équipée d'une sonde de température 129 pour mesurer la température du milieu à proximité du capteur. En variante, on mesure la température du sédiment au niveau du capteur pour déterminer la correction à apporter. En pratique, la température à l'intérieur de la tige et à l'extérieur de la tige tend à s'équilibrer, ce qui fait que la mesure de la température de l'huile revient à mesurer la température du sédiment.

La correction $\Delta P$ à apporter à la mesure de pression est la suivante:

$$\Delta P = \rho_{eau}*g*h - \rho_{huile}*g*h = (\rho_{eau} - \rho_{huile})*g*h$$

Où

- $\rho_{huile}$ désigne la masse volumique de l'huile pour une température et une pression données;
- $\rho_{eau}$ désigne la masse volumique de l'eau de mer pour une température et une pression données;
- h désigne la distance entre le capteur et l'interface eau / sédiment.

Pour une mesure effectuée à 50 mètres sous l'interface eau/sédiment à 15 °C, à faible profondeur, la correction $\Delta P$ à apporter est égale à:

$$\Delta P = g*h*(\rho_{eau} - \rho_{huile}) = 9,81*50*(1,029-0,851) = 87,3 \text{ kPa}.$$

Cette valeur $\Delta P$ est à additionner à la valeur mesurée par le capteur.

Un capteur de mesure de pression relative présente un encombrement réduit par rapport à un capteur de mesure différentielle. L'utilisation d'un tel capteur permet à la fois de supprimer la partie en eau présente dans les systèmes de l'art antérieur et réduire le diamètre de la tige pour qu'elle soit proche des diamètres de tige exploitables par le pénétromètre Penfeld.

Le mode de réalisation décrit ci-dessus est donné à titre d'exemple. Il est évident pour l'homme de l'art qu'il peut être modifié, notamment quant à la position des capteurs de pression, des capteurs de température et des cartes électroniques associées. Ces éléments peuvent par exemple être déportés dans les tronçons de tube 120.

## Revendications

1. Procédé de détermination de la pression interstitielle dans le sédiment marin à différentes profondeurs, ledit procédé comportant l'étape suivante:

   - enfoncer dans le sédiment marin une tige (102) creuse équipée d'une pluralité de capteurs de pression (124) et de capteurs de température (129) disposés le long de la tige,

   **caractérisé en ce que**, ladite tige étant remplie d'un liquide diélectrique, le dit procédé comporte en outre les étapes suivantes :

   - mesurer au moyen des capteurs de pression la pression dans le sédiment par rapport à la pression du liquide diélectrique dans la tige pour une pluralité de niveaux de profondeur,
   - déterminer au moyen des capteurs de température la température à proximité des capteurs de pression, et

- déterminer une valeur réelle de pression interstitielle pour chacun des niveaux de profondeur à partir des mesures de pression fournies par les capteurs de pression et les mesures de température fournies par les capteurs de température.

2. Procédé selon la revendication 1, **caractérisé en ce que** les capteurs de température sont présents à l'intérieur de la tige pour mesurer la température du liquide diélectrique au niveau de chaque capteur de pression.

3. Procédé selon la revendication 1 ou 2, **caractérisé en ce que** la tige est enfoncée dans le sédiment marin à l'aide d'un pénétromètre à tambour, ladite tige étant enroulée autour du tambour avant enfoncement.

4. Procédé selon l'une quelconque des revendications précédentes, **caractérisé en ce que** le liquide diélectrique est de l'huile.

5. Dispositif de mesure de pression interstitielle dans le sédiment marin, ledit dispositif comprenant:

   - une tige (102) creuse destinée à être enfoncée dans le sédiment marin,
   - une pluralité de capteurs de pression (124) disposés le long de la tige pour mesurer la pression interstitielle dans le sédiment marin à différentes profondeurs,
   - une pluralité de capteurs de température (129) disposés à proximité des capteurs de pression pour mesurer la température à proximité des capteurs de pression,
   - une pluralité de cartes (125) d'acquisition des mesures connectées chacune à un capteur de pression et à un capteur de température, et un bus numérique de communication (126) pour transmettre les données de mesure vers l'extérieur de la tige,

   **caractérisé en ce que** la tige (102) est remplie de liquide diélectrique (H) et les capteurs de pression sont des capteurs relatifs agencés dans la tige pour chacun mesurer, pour une profondeur donnée, la pression dans le sédiment par rapport à la pression de du liquide diélectrique dans la tige et **en ce que** ladite pluralité de cartes d'acquisition de mesure et le bus numérique de communication sont disposés à l'intérieur de la tige remplie de liquide diélectrique.

6. Dispositif selon la revendication 5, **caractérisé en ce que** les capteurs de température sont présents à l'intérieur de la tige pour mesurer la température du liquide diélectrique au niveau de chaque capteur de pression.

7. Dispositif selon la revendication 5 ou 6, **caractérisé en ce que** la tige comporte une pluralité de tronçons (120) assemblés entre eux par des éléments de liaison (121) étanches.

8. Dispositif selon la revendication 7, **caractérisé en ce que** les tronçons de tube et les éléments de liaison sont réalisés en un matériau ductile, tel que de l'acier inoxydable X2CrNiMo 17-12-2.

9. Dispositif selon la revendication 7 ou 8, **caractérisé en ce que** la tige comporte un capteur de pression et un capteur de température par tronçon de tube ou par élément de liaison.

10. Dispositif selon l'une quelconque des revendications 5 à 9, **caractérisé en ce que** le liquide diélectrique est de l'huile.

11. Système de mesure de pression interstitielle dans le sédiment marin à différents niveaux de profondeur, comprenant un dispositif selon l'une quelconque des revendications 5 à 10,
**caractérisé en ce qu'**il comporte en outre un pénétromètre (101) pour enfoncer la tige dans le sédiment marin (S), ledit pénétromètre comprenant un tambour (104) autour duquel est enroulée la tige avant enfoncement et des moyens d'enfoncement (105) de la tige dans le sédiment marin, la tige étant apte à être au moins une fois enroulée par cintrage autour du tambour et déroulée du tambour et lesdits moyens d'enfoncement étant aptes à dérouler et redresser mécaniquement la tige du tambour puis l'enfoncer dans le sédiment marin.

**Patentansprüche**

1. Verfahren zur Bestimmung des Porendrucks im Meeressediment in unterschiedlichen Tiefen, wobei das Verfahren den folgenden Schritt umfasst:

- Eintreiben einer Hohlstange (102), die mit einer Vielzahl von entlang der Stange angeordneten Drucksensoren (124) und Temperatursensoren (129) ausgestattet ist, in das Meeressediment,

**dadurch gekennzeichnet, dass**, wenn die Stange mit einer dielektrischen Flüssigkeit gefüllt ist, das Verfahren weiter die folgenden Schritte umfasst:

- Messen, mittels der Drucksensoren, des Drucks im Sediment im Verhältnis zum Druck der dielektrischen Flüssigkeit in der Stange über eine Vielzahl von Tiefenbereichen,
- Bestimmen, mittels der Temperatursensoren, der Temperatur in der Nähe der Drucksensoren, und
- Bestimmen eines realen Porendruckwerts für jeden der Tiefenbereiche auf Grundlage der von den Drucksensoren gelieferten Druckmessungen und der von den Temperatursensoren gelieferten Temperaturmessungen.

2. Verfahren nach Anspruch 1, **dadurch gekennzeichnet, dass** die Temperatursensoren im Inneren der Stange vorliegen, um die Temperatur der dielektrischen Flüssigkeit im Bereich jedes Drucksensors zu messen.

3. Verfahren nach Anspruch 1 oder 2, **dadurch gekennzeichnet, dass** die Stange mithilfe eines Trommel-Penetrometers in das Meeressediment eingetrieben wird, wobei die Stange vor dem Eintreiben um die Trommel gewickelt ist.

4. Verfahren nach einem der vorstehenden Ansprüche, **dadurch gekennzeichnet, dass** die dielektrische Flüssigkeit Öl ist.

5. Vorrichtung zum Messen von Porendruck im Meeressediment, wobei die Vorrichtung umfasst:

- eine Hohlstange (102), die dazu bestimmt ist, in das Meeressediment eingetrieben zu werden,
- eine Vielzahl von entlang der Stange angeordneten Drucksensoren (124), um den Porendruck im Meeressediment in unterschiedlichen Tiefen zu messen,
- eine Vielzahl von Temperatursensoren (129), die in der Nähe der Drucksensoren angeordnet sind, um die Temperatur in der Nähe der Drucksensoren zu messen,
- eine Vielzahl von Karten (125) zur Erfassung der Messungen, die jede mit einem Drucksensor und mit einem Temperatursensor verbunden sind, und einen digitalen Kommunikationsbus (126), um die Messdaten zur Außenseite der Stange zu übertragen,

**dadurch gekennzeichnet, dass** die Stange (102) mit dielektrischer Flüssigkeit (H) gefüllt ist und die Drucksensoren Relativsensoren sind, die in der Stange eingerichtet sind, um jeder für eine gegebene Tiefen den Druck im Sediment im Verhältnis zum Druck der dielektrischen Flüssigkeit in der Stange zu messen, und dadurch, dass die Vielzahl von Messerfassungskarten und der digitale Kommunikationsbus im Inneren der mit dielektrischer Flüssigkeit gefüllten Stange angeordnet sind.

6. Vorrichtung nach Anspruch 5, **dadurch gekennzeichnet, dass** die Temperatursensoren im Inneren der Stange vorliegen, um die Temperatur der dielektrischen Flüssigkeit im Bereich jedes Drucksensors zu messen.

7. Vorrichtung nach Anspruch 5 oder 6, **dadurch gekennzeichnet, dass** die Stange eine Vielzahl von Abschnitten (120) umfasst, die mit dichten Verbindungselementen (121) aneinandergefügt sind.

8. Vorrichtung nach Anspruch 7, **dadurch gekennzeichnet, dass** die Rohrabschnitte und die Verbindungselemente aus einem duktilen Material wie etwa X2CrNiMo 17-12-2-Edelstahl ausgeführt sind.

9. Vorrichtung nach Anspruch 7 oder 8, **dadurch gekennzeichnet, dass** die Stange einen Drucksensor und einen Temperatursensor pro Rohrabschnitt oder pro Verbindungselement umfasst.

10. Vorrichtung nach einem der Ansprüche 5 bis 9, **dadurch gekennzeichnet, dass** die dielektrische Flüssigkeit Öl ist.

11. System zum Messen von Porendruck im Meeressediment in unterschiedlichen Tiefenbereichen, das eine Vorrichtung nach einem der Ansprüche 5 bis 10 umfasst, **dadurch gekennzeichnet, dass** es weiter ein Penetrometer (101) umfasst, um die Stange in das Meeressediment (S) einzutreiben, wobei das Penetrometer eine Trommel (104), um die die Stange vor dem Eintreiben gewickelt ist, und Mittel zum Eintreiben (105) der Stange in das Meeressediment umfasst, wobei die Stange mindestens einmal durch Biegen um die Trommel gewickelt und von der Trommel abgewickelt werden kann, und wobei die Eintreibemittel die Stange von der Trommel abwickeln und mechanisch geraderichten und dieselbe anschließend in das Meeressediment eintreiben können.

**Claims**

1. Method of determining the interstitial pressure in the marine sediment at various depths, said method comprising the following step:

   - sinking into the marine sediment a hollow rod (102) equipped with a plurality of pressure sensors (124) and temperature sensors (129) disposed along the rod,

   **characterised in that**, said rod being filled with a dielectric liquid, said method further comprises the following steps:

   - measuring by means of the pressure sensors the pressure in the sediment with respect to the pressure of the dielectric liquid in the rod for a plurality of depth levels,
   - determining by means of the temperature sensors the temperature in proximity to the pressure sensors, and
   - determining a real value of interstitial pressure for each of the depth levels on the basis of the pressure measurements provided by the pressure sensors and the temperature measurements provided by the temperature sensors.

2. Method according to claim 1, **characterised in that** the temperature sensors are present inside the rod for measuring the temperature of the dielectric liquid on each pressure sensor.

3. Method according to claim 1 or 2, **characterised in that** the rod is sunk into the marine sediment using a drum penetrometer, said rod being wound around the drum before sinking.

4. Method according to any preceding claim, **characterised in that** the dielectric liquid is oil.

5. Device for measuring interstitial pressure in the marine sediment, said device comprising:

   - a hollow rod (102) intended to be sunken in the marine sediment,
   - a plurality of pressure sensors (124) disposed along the rod for measuring the interstitial pressure in the marine sediment at various depths,
   - a plurality of temperature sensors (129) disposed in the vicinity of the pressure sensors for measuring the temperature in the vicinity of the pressure sensors,
   - a plurality of measurement acquisition cards (125) each connected to a pressure sensor and to a temperature sensor, and a digital communication bus (126) for transmitting the measurement data to the outside of the rod,

   **characterised in that** the rod (102) is filled with dielectric liquid (H) and the pressure sensors are relative sensors arranged in the rod for each to measure, for a given depth, the pressure in the sediment with respect to the pressure of the dielectric liquid in the rod and **in that** said plurality of measurement acquisition cards and the digital communication bus are disposed inside the rod filled with dielectric liquid.

6. Device according to claim 5, **characterised in that** the temperature sensors are present inside the rod for measuring the temperature of the dielectric liquid on each pressure sensor.

7. Device according to claim 5 or 6, **characterised in that** the rod comprises a plurality of segments (120) assembled together by sealed connecting elements (121).

8. Device according to claim 7, **characterised in that** the tube segments and the connecting elements are made from a ductile material, such as X2CrNiMo 17-12-2 stainless steel.

9. Device according to claim 7 or 8, **characterised in that** the rod comprises a pressure sensor and a temperature sensor per tube segment or per connecting element.

10. Device according to any of claims 5 to 9, **characterised in that** the dielectric liquid is oil.

**11.** System for measuring interstitial pressure in the marine sediment at different depth levels, comprising a device according to any of claims 5 to 10,
**characterised in that** it further comprises a penetrometer (101) for sinking the rod in the marine sediment (S), said penetrometer comprising a drum (104) around which is wound the rod before sinking and means for sinking (105) the rod in the marine sediment, the rod being able to be at least wound once by bending around the drum and unwound from the drum and said means of sinking being able to unwind and mechanically rectify the rod of the drum then sink it in the marine sediment.

FIG.1
(Art antérieur)

FIG.2
(Art antérieur)

FIG.3

(Art antérieur)

FIG.4

FIG.5

vers module de stockage des données

FIG.6

FIG.7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **DE J.MEUNIER ; N.SULTAN ; P.JÉGOU ; F.HARMEGNIES.** Le pénétromètre Penfeld. *SeatechWeek,* 2004